# EUROPÄISCHE PATENTSCHRIFT

(11) **EP 4 041 433 B1**
(45) Veröffentlichungstag und Bekanntmachung des Hinweises auf die Patenterteilung: **06.05.2026**
(21) Anmeldenummer: 20793250.0
(22) Anmeldetag: 09.10.2020
(51) Int. Cl.: B01D 47/06, F24F 6/16, F24F 13/078

(54) **VORRICHTUNG ZUR REINIGUNG VON GAS**
APPARATUS FOR CLEANING GAS
DISPOSITIF D'ÉPURATION DE GAZ

(30) Priorität: 09.10.2019 AT 508612019
(43) Veröffentlichungstag der Anmeldung: 17.08.2022
(73) Patentinhaber: Buchegger, Andreas, 5023 Salzburg (AT)
(72) Erfinder: Buchegger, Andreas, 5023 Salzburg (AT)
(74) Vertreter: Babeluk Patentanwälte GmbH
(86) Internationale Anmeldenummer: PCT/AT2020/060362
(87) Internationale Veröffentlichungsnummer: WO 2021/068021

(56) Entgegenhaltungen:
- AT-B- 367 894
- CN-A- 107 131 588
- FR-A1- 3 060 717
- KR-A- 20170 025 141
- KR-A- 20170 051 276
- KR-B1- 101 626 256
- US-A1- 2006 102 001

## Beschreibung

Die Erfindung betrifft eine Vorrichtung zur Reinigung von Gas, gemäß Anspruch 1, insbesondere von Luft, mit einem Oberteil und einem Unterteil, die miteinander in lösbarer Verbindung stehen, wobei in dem Unterteil ein zentraler, vertikal angeordneter Lufteinlaufstutzen für verunreinigte Luft vorgesehen ist, sowie ein Flüssigkeitsbehälter für eine Reinigungsflüssigkeit mit einer Düseneinrichtung, und in dem Oberteil befindet sich eine Luftansaugeinrichtung sowie eine Einrichtung zur Bildung zumindest eines horizontalen Flüssigkeitsfilms und eine in dem Oberteil angeordnete Luftumlenkeinrichtung, die die angesaugte Luft durch den Flüssigkeitsfilm leitet, vorgesehen ist. Des Weiteren betrifft die Erfindung ein Verfahren zur Luftreinigung gemäß Anspruch 14.

Es sind zahlreiche Vorrichtungen zur Reinigung und/oder Befeuchtung von Luft bekannt geworden. So offenbart beispielsweise die US 2006/0097411 A1 ein Gerät zur Luftreinigung mit einem zylindrischen Gehäuse mit deckelseitigen Lufteinlässen und einer Gebläseeinheit mit Sprühkopf, wobei die Reinigungsflüssigkeit, hier Wasser, über den Sprühkopf in die angesaugte Luft eingebracht wird und diese durch Adsorption der Verunreinigungen säubert.

Die DE 196 19 885 A1 zeigt eine Luftreinigungs- und Luftbefeuchtungseinrichtung, in der der Luftstrom mehrfach durch eine Wassersäule gedrückt wird.

Die KR 2017 0025141 A1 offenbart eine Vorrichtung, bei der ein Luftstrom durch einen horizontalen Wasserfilm geführt wird und damit gereinigt wird. Danach wird der Luftstrom über Auslasskanäle 22 nach außen geleitet. Dies ist wenig vorteilhaft, da so ein großer Teil des Wassers mit dem Luftstrom in die Umgebungsluft geführt wird, teilweise auch in Form von noch nicht verdampften Wassertröpfchen. Das kann dazu führen, dass sich die Wassertröpfchen in der Umgebung der Vorrichtung absetzen und so Oberflächen ungewollte befeuchten können. Andererseits wird dadurch sehr viel Wasser verbraucht.

Nachteilig an diesen Vorrichtungen ist, dass sie voluminös sind und häufig für den Einsatz am Boden aufgestellt werden müssen. Dabei verbrauchen sie Platz und stellen häufig unerwünschte Hindernisse in den Räumen und Hallen dar.

Die AT 367 894 schließlich beschreibt eine Vorrichtung zur Reinigung, Befeuchtung und Ionisierung von Luft der eingangs erwähnten Art mit einem Wassertank, aus welchem einem Schleuderrad Wasser zugeführt und in einer horizontalen Schicht verteilt wird, wobei zwischen dem Rand einer das Schleuderrad umgebenden, nach unten offenen Wanne und dem Rand des Schleuderrads einerseits und der Außenwand des Wassertanks andererseits jeweils ein kreisringförmiger Durchtrittsquerschnitt für den von einem Lüfter nach unten durch die Wasserschicht gelenkten und anschließend wieder aufsteigenden Luftstrom gebildet ist, wobei sich der obere Rand der Wanne geringfügig oberhalb des vom Außenrand des Schleuderrades abgegebenen Wasserfilm befindet und der Wasserfilm auf die den äußeren Durchtrittsquerschnitt der Luft begrenzende Wand des Wassertanks, die in die Ebene des Wasserfilm nach innen abgewinkelt ist, auftrifft.

Es ist daher Aufgabe der Erfindung, eine Vorrichtung der eingangs erwähnten Art bereitzustellen, die eine verbesserte Reinigungsleistung im Vergleich zum Stand der Technik aufweist und gleichzeitig kompakt und platzsparend ausgeführt ist.

Diese Aufgabe wird erfindungsgemäß dadurch gelöst, dass in einem äußeren Randbereich der Vorrichtung zwischen Oberteil und Unterteil zumindest eine Abtropfeinrichtung vorgesehen sind, deren Abtropfelemente in einem Winkel von maximal 160° zu dem horizontalen Flüssigkeitsfilm ausgerichtet sind. Bei der vorliegenden Erfindung wird der Luftstrom über einen Lufteinlaufstutzen angesaugt und derart umgelenkt, dass er durch den horizontalen Flüssigkeitsfilm hindurchtritt. Dabei werden einerseits bereits teilweise Verunreinigungen in der Luft wie beispielsweise Staub, Feinstaub und Ultrafeinstaub an die Flüssigkeit abgegeben; andererseits werden aber auch Flüssigkeitsteilchen von der Luft mitgerissen. Diese Flüssigkeitsteilchen absorbieren den Großteil der in der Luft enthaltenen Verunreinigungen und werden beim Durchtritt der feuchten Luft an der Abtropfeinrichtung abgeschieden, sodass sie gemeinsam mit in ihr noch enthaltenen Verunreinigungen aus der Luft entfernt werden, bevor die gereinigte Luft die erfindungsgemäße Vorrichtung verlässt. Die im Winkel von maximal 160°, vorzugsweise zwischen 140° und 130°, besonders vorzugsweise zwischen 135° und 130°, angeordneten Abtropfelemente der Abtropfeinrichtung erlauben einen besonders intensiven Kontakt der Luft mit der Abtropfeinrichtung, wodurch eine effiziente Abscheidung der Flüssigkeit und damit der Verunreinigungen stattfindet (selbstreinigende Funktion). Des Weiteren wird die Luft dabei entschleunigt. Dabei kann vorgesehen sein, dass die Abtropfelemente im Wesentlichen senkrecht zum Flüssigkeitsfilm stehen. Gleichzeitig wird durch die Anordnung der Abtropfeinrichtung im Randbereich der erfindungsgemäßen Vorrichtung eine besonders flache und kompakte Bauweise erzielt, die es erlaubt, die Vorrichtung beispielsweise an der Decke eines Raumes zu befestigen oder von der Decke abzuhängen. Im Stand der Technik wird die zu reinigende, befeuchtete Luft um Kanten umgeleitet, sodass es in diesen Kantenbereichen zu vermehrter Tropfenbildung kommt, die sich störend auf die Reinigungswirkung der bekannten Vorrichtungen auswirkt. Bei der vorliegenden Erfindung wird der Luftstrom nach dem Austritt aus der Luftansaugeinrichtung im Wesentlichen horizontal zwangsgeführt, ohne dass hierbei Kanten in den Luftstrom ragen, sodass insbesondere auch keine unerwünschte Geräuschentwicklung wie beispielsweise Pfeifen auftritt.

Die Abtropfeinrichtung kann dabei gleichzeitig als Schalldämpfer wirken und die Schallbelastung des Raumes durch die Vorrichtung zu minimieren.

Vorzugsweise ist eine Pumpeinrichtung vorgesehen, die die Reinigungsflüssigkeit aus dem Flüssigkeitsbehälter entnimmt, zur Düseneinrichtung pumpt und damit einen ausreichenden Druck für die Düseneinrichtung sicherstellt. Alternativ dazu könnte auch vorgesehen sein, den Wasserdruck einer Frisch- oder Brauchwasserleitung zu nutzen und mittels Druckminderer (vorzugsweise angesteuert) die benötigte Wassermenge Drehzahlabhängig zuzuführen.

Um einen gleichmäßigen horizontalen Flüssigkeitsfilm zu erhalten, weist die Einrichtung zur Bildung zumindest eines horizontalen Flüssigkeitsfilms bevorzugterweise zumindest ein an der dem Lufteinlaufstutzen zugewandten Seite der Luftansaugeinrichtung angeordnetes Ansaugstück auf, das bei Rotation der Luftansaugeinrichtung ebenfalls rotiert. Das rotierende Ansaugstück ist hierbei beabstandet von dem am Unterteil fixierten Lufteinlaufstutzen angeordnet, wodurch ein schmaler Spalt von geringer Breite zwischen Lufteinlaufstutzen und Ansaugstück gebildet wird.

Es kann auch vorgesehen sein, dass mehrere, vorzugsweise zwei Ansaugstücke vorgesehen sein, welche bei Rotation der Luftansaugeinrichtung ebenfalls rotieren und jeweils einen horizontalen Flüssigkeitsfilm erzeugen. Dadurch muss die Luft mehrere Passagen durch die Flüssigkeit durchmachen und wird verstärkt gereinigt.

Die Flüssigkeit wird mittels einer Pumpe oder durch eine Frischwasserleitung, gegebenenfalls mittels Druckminderer, und einer Düseneinrichtung auf die Schaufeln des rotierenden Ansaugstücks aufgebracht, wobei aufgrund der herrschenden Fliehkräfte die Flüssigkeit beschleunigt und über den oberen Austrittsspalt fortgeschleudert wird, und sich bei einem geeigneten Pumpendruck ein gleichmäßiger horizontaler, radial von dem Ansaugstück ausgehender Flüssigkeitsfilm bildet.

Bevorzugterweise entspricht dabei der Durchmesser des dem Ansaugstück zugewandten Endes jenem des Lufteinlaufstutzens und verbreitert sich in Richtung Luftansaugeinrichtung, wobei die Kontur der Außenfläche von Ansaugstück im Wesentlichen einem Hyperbelast (im Schnitt) entspricht. Die Form des Lufteinlaufstutzens ist vorzugsweise Kegelstumpfförmig ausgebildet und verjüngt sich in Richtung Luftansaugeinrichtung. Der horizontale Flüssigkeitsfilm entspringt vorzugsweise an der Kante zwischen Luftansaugeinrichtung und Ansaugstück, also vorzugsweise an der konkaven Seite eines Schleuderrads, wo sein Durchmesser am größten ist.

Eine besonders kompakte Bauweise wird erzielt, wenn der Flüssigkeitsbehälter im Unterteil um den Lufteinlaufstutzen spitz zusammenläuft. Dadurch wird die Vorrichtung besonders flach, weil kein eigener platzraubender Bauteil als Flüssigkeitsbehälter fungiert, sondern dieser in dem Unterteil der erfindungsgemäßen Vorrichtung integriert ist. Dabei ist mit spitz zusammenlaufend gemeint, dass sich zumindest eine Kante ergibt, in der sich zwei Flächen unterschiedlicher Steigungen treffen. Alternativ kann auch der Unterteil kreisringförmig um den Lufteinlaufstutzen angeordnet oder geschwungen ausgeführt sein.

Als besonders geeignet in ihrer Funktion als Luftansaugeinrichtung haben sich Radiallüfter herausgestellt. Die Reinigungsleistung der erfindungsgemäßen Vorrichtung ist hierbei abhängig von der Menge der angesaugten Luft pro Zeiteinheit, die wiederum über die Leistung der Luftansaugeinrichtung steuerbar ist.

Um die angesaugte Luft durch den Flüssigkeitsfilm hindurchzuleiten, verfügt die Luftumlenkeinrichtung in einer bevorzugten Ausführung der Erfindung über zu dem horizontalen Flüssigkeitsfilm hinabfallende, vorzugsweise trimmbare Leitbleche oder einem Trimmring, die oder der von der Luftansaugeinrichtung minimal beabstandet ist. Dadurch wird die Luft durch den Flüssigkeitsfilm zwangsgeführt und kommt so in intensiven Kontakt mit der Flüssigkeit, ohne um Kanten umgeleitet zu werden.

Für eine erhöhte Reinigungskapazität der Erfindung haben sich Abtropfelemente der Abtropfeinrichtung erwiesen, die aus einer Vielzahl von Borsten bestehen. Durch den geringen Durchmesser der Borsten können auf geringen Raum eine große Anzahl, beispielsweise 10.000 und mehr angeordnet werden, wodurch eine sehr große Oberfläche für die Flüssigkeitsabscheidung angeboten wird. Vorzugsweise weisen die Borsten zumindest teilweise eine elektrisch leitende Oberfläche auf und besonders vorzugsweise sind diese zumindest teilweise miteinander elektrisch leitend verbunden. Die verbessert das Abtropfverhalten der Flüssigkeit.

Bevorzugterweise steht die Abtropfeinrichtung mit dem Flüssigkeitsbehälter in Verbindung, sodass die in der Abtropfeinrichtung abgeschiedene, die Verunreinigungen enthaltende Flüssigkeit in den Flüssigkeitsbehälter zurückfließt. Die so rückgewonnene Flüssigkeit kann abgeführt oder erneut in einen Kreislauf geführt werden. Alternativ dazu kann ein eigener Auffangbehälter für die verunreinigte Flüssigkeit vorgesehen sein.

Weiters ist vorteilhaft, wenn die Abtropfeinrichtung zumindest eine Leitfläche aufweist, welche entlang einer Hauptströmungsrichtung eine geschwungene Form aufweist, wobei sich die Leitfläche entlang der Hauptströmungsrichtung von vom Gasstrom weg biegt. Die Leitfläche ist vorzugsweise auf dem Oberteil angeordnet. Die Hauptströmungsrichtung ergibt sich aus der Richtung, in die das Gas hauptsächlich strömt. Dabei kann auch vorgesehen sein, dass die Abtropfeinrichtung zumindest eine Leitfläche aufweist, welche entlang einer Hauptströmungsrichtung eine geschwungene Form mit aufweist, wobei die Steigung der Leitfläche in Bezug zur Hauptströmungsrichtung zumindest im von der Luftansaugeinrichtung weiter entfernten Bereich entlang der Hauptströmungsrichtung stetig zunimmt. Durch das Entlangführen des vorbeiströmenden Gases wird das Entstehen von Wirbeln verhindert und so die Geräuschbelastung durch die Vorrichtung gesenkt. Dabei erstreckt sich die Leitfläche vorzugsweise ringförmig um die Luftansaugeinrichtung herum. Durch die nach außen immer steiler werdende Leitfläche ergibt sich eine bauchige, kreisförmige oder parabelastförmige Form, die besonders vorteilhaft ist. Vorzugsweise ist die Leitfläche im Bereich eines Gasausgangs zum Abführen des gereinigten Gases aus der Vorrichtung angeordnet.

In einer besonders günstigen Ausführung der Erfindung ist die Flüssigkeit Wasser. Wasser ist bekannt für seine hohe Absorptionsfähigkeit von Verunreinigungen aus der Luft und kann im Normalfall problemlos entsorgt werden. In anderen Fällen kann es jedoch angebracht sein, dass eine andere Reinigungsflüssigkeit, beispielsweise ein Lösungsmittel anstelle von Wasser zum Einsatz kommt. Hier ist dann die intensive Abscheidungsrate in der erfindungsgemäßen Abtropfeinrichtung besonders von Vorteil.

Die Aufgabe wird erfindungsgemäß auch durch ein Verfahren zur Reinigung von Luft unter Verwendung der erfindungsgemäßen Vorrichtung gelöst, wobei eine Flüssigkeit aus einem Flüssigkeitsbehälter horizontal verteilt wird, die zu reinigende Luft zumindest einmal durch den horizontalen Flüssigkeitsfilm hindurchgeleitet wird, die Luft nach dem Durchtritt durch den Flüssigkeitsfilm über eine Abtropfeinrichtung geführt wird, wobei die in der Luft befindliche, mit Verunreinigungen der Luft angereicherte Flüssigkeit an in einem Winkel von maximal 160° zu dem horizontalen Flüssigkeitsfilm angeordneten Abtropfelementen der Abtropfeinrichtung abgeschieden wird.

Besonders vorteilhaft ist, wenn die Vorrichtung zumindest eine UV-Lichtquelle zur Bestrahlung der Luft aufweist. Die UV-Lichtquelle ist dabei eine Lichtquelle, welche Licht im UV-Bereich erzeugen kann, also Licht mit Wellenlängen im Bereich von etwa 100 nm bis 380 nm. Dabei kann die Lichtquelle auch Licht in anderen Wellenlängenbereichen produzieren. Vorzugsweise ist durch die UV-Lichtquelle Licht im UV-C Bereich erzeugbar, also Licht in einem Wellenlängenbereich von etwa 100 nm bis 280 nm. Dadurch können Keime in dem Gas, welches bestrahlt und gereinigt wird, zusätzlich abgetötet werden und so die Keimzahl verringert werden oder das Gas sogar sterilisiert werden. Besonders vorzugsweise ist die UV-Lichtquelle dazu ausgebildet, Hopsitalismuskeime abzutöten, also Keime, welche insbesondere wegen des Risikos schlecht behandelbarer nosokomialer Infektionen in Gesundheitseinrichtungen problematisch sind. Die UV-Lichtquelle kann dazu eingerichtet sein, weitere Teile der Vorrichtung oder das Wasser zu bestrahlen und an diesen Teilen ebenso eine Keimreduktion oder Sterilisation zu erreichen.

Weiters ist vorteilhaft, wenn eine die UV-Lichtquelle umfassende Bestrahlungseinheit an der, dem Oberteil abgewandten Seite des Unterteils angeordnet ist. Damit wird vorzugsweise die eingesaugte Luft vor der Reinigung keimreduziert. Wird die Luft auf der Seite des Oberteils angesaugt, kann vorgesehen sein, dass eine die UV-Lichtquelle umfassende Bestrahlungseinheit an der, dem Unterteil abgewandten Seite des Oberteils angeordnet ist.

Weiters ist vorteilhaft, wenn die Bestrahlungseinheit eine gasdurchlässige, UV-lichtdichte Filterwand aufweist. Damit kann die Filterwand eine Grobfilterung durchführen und verhindern, dass das Licht nach außen dringt. Die Filterwand kann beispielsweise ein Fließ sein. Dabei ist vorzugsweise vorgesehen, dass die Innenseite der Filterwand von der UV-Lichtquelle vollständig ausgeleuchtet wird, um das Entstehen von Keimherden zu vermeiden.

Im Folgenden wird anhand von nicht-einschränkenden Ausführungsbeispielen mit zugehörigen Figuren die Erfindung näher erläutert. Darin zeigt
- Fig. 1: eine Schnittansicht einer erfindungsgemäßen Vorrichtung zur Reinigung von Luft;
- Fig. 2a: eine seitliche Darstellung des Radiallüfters mit Ansaugstück;
- Fig. 2b: ein vergrößerter Ausschnitt aus Fig. 2a;
- Fig. 3: eine Seitenansicht des Radiallüfters; und
- Fig 4: eine schematische Seitendarstellung einer zweiten Ausführungsform ohne Oberteil und Abtropfeinrichtung;
- Fig. 5: einen Schnitt durch eine dritte Ausführungsform mit Bestrahlungseinheit;
- Fig. 6: eine Seitenansicht der dritten Ausführungsform;
- Fig. 7: und Fig. 9 einen Schnitt und eine Seitenansicht einer alternativen Ausführungsform des Radiallüfters mit Ansaugstück; und
- Fig. 8: und Fig. 10 sind vergrößerte Ausschnitte U und V aus den Figuren 7 und 9.

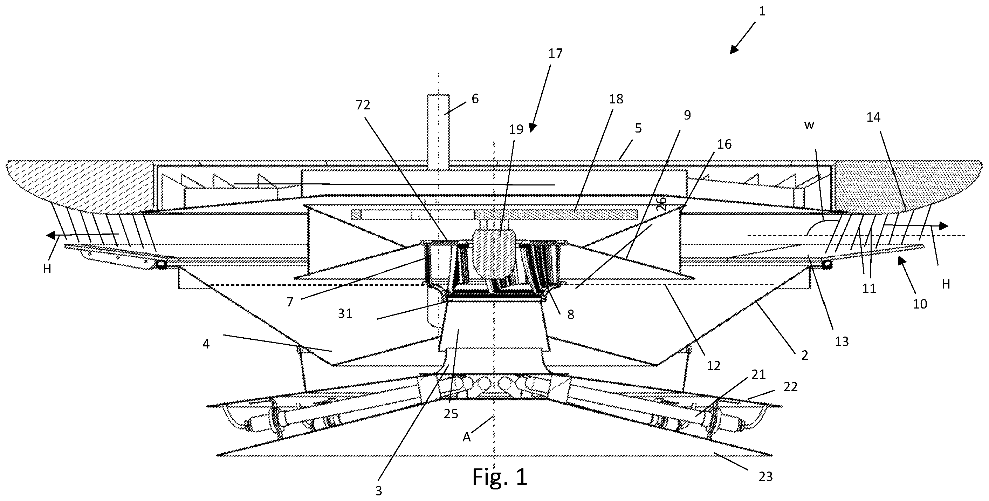

Gemäß der Figur 1 besteht die erfindungsgemäße Vorrichtung 1 zur Reinigung, Befeuchtung und Ionisierung von Luft aus einem wannenförmigen Unterteil 2, vorzugsweise aus Niro Stahlblech, in dessen Zentrum ein Lufteinlaufstutzen 3 angeordnet ist, über den die verunreinigte Luft aus der Umgebung angesaugt wird. Allgemein besitzt die Vorrichtung 1, die üblicherweise an der Decke befestigt oder abgehängt wird, eine im Wesentlichen kreisrunden Grundfläche. Die Ausführungsform ist drehsymmetrisch um eine Mittelachse A.

Um den Lufteinlaufstutzen 3 herum weist der Unterteil 2 eine spitz zusammenlaufende Vertiefung auf, die als Flüssigkeitsbehälter 4 fungiert, dessen maximales Flüssigkeitsniveau unterhalb der oberen Kante 31 des Lufteinlaufstutzens 3 liegt.

Ein oberhalb des Unterteils 2 angeordnet Oberteil 5 der Vorrichtung 1 besitzt im Wesentlichen den gleichen Außendurchmesser wie der Unterteil 2, sein Innendurchmesser (das ist der Durchmesser einer zentrisch angeordnete Öffnung 17 im Oberteil 5) ist kleiner als der Außendurchmesser der Luftumlenkeinrichtung 9. Die Luftumlenkeinrichtung 9 ist an den Befestigungshülsen 6 (Säulen) lösbar fixiert und liegt innerhalb der Abtropfeinrichtung. Eine runde Verbindungskante zwischen Luftumlenkeinrichtung 9 und Oberteil 5 ist über einen Gummidichtring 16 abgedichtet. Im Zentrum des Oberteils 5 ist eine Luftansaugeinrichtung 7, beispielsweise ein Radiallüfter, angeordnet, der an seiner dem Unterteil 2 zugewandten Seite ein Ansaugstück 8 aufweist. Die Luftansaugeinrichtung 7 ist über ein Halterad 18 mit der Luftumlenkeinrichtung 9 verbunden und wird über dieses gehalten. Ein Motor 19 im Zentrum der Luftansaugeinrichtung 7 dreht diese und ist über das Halterad abgestützt. Dieses Ansaugstück 8, das aufgrund der Rotationsbewegung der Luftansaugeinrichtung 7 ebenfalls rotiert, ist oberhalb des Lufteinlaufstutzens 3 angeordnet, wobei zwischen dem statischen Lufteinlaufstutzen 3 und dem rotierenden Ansaugstück 8 ein schmaler Spalt, beispielsweise mit einer Breite von etwa 0,5 mm, verbleibt, um die Rotationsbewegung des Ansaugstückes 8 nicht zu beeinträchtigen. Lufteinlaufstutzen 3 und Ansaugstück 8 weisen auf der Höhe, an der sie sich treffen, den gleichen Innendurchmesser auf, wobei das Ansaugstück 8 im Schnitt eine hyperbelastähnliche Form aufweist. Lufteinlaufstutzen 3 weist eine konvergent-divergente (also eine kegelstumpfartige) Form auf. Diese Form verhindert die Bildung von unerwünschten Pfeifgeräuschen insbesondere bei der Kante 31 beim Ansaugen der Luft.

Unterhalb des Oberteils 5 ist des Weiteren eine Luftumlenkeinrichtung 9 in Form eines Trimmrings in der vorliegenden Ausführung der Erfindung in Form von radial um die Luftansaugeinrichtung 7 angeordnet. Zwischen Oberteil 5 und Unterteil 2 schließlich befindet sich im Randbereich der Vorrichtung 1 eine Abtropfeinrichtung 10, auch Wasserkat genannt, die eine Vielzahl von geneigt ausgeführten Abtropfelementen 11 in Form von Borsten aufweist. Die Borsten sind beispielsweise aus Nirosta gefertigt, besitzen einen Durchmesser von 1,2 mm und sind in Achsabständen von 8 mm in eine Styrodurplatte gesteckt. Die Abtropfelemente 11 sind auf einer Leitfläche 14 angeordnet und von Oberfläche 13 ca. 1 cm beabstandet.

Die Leitfläche 14 ist ringförmig um die Luftumlenkeinrichtung 9 und am Oberteil 5 angeordnet. Sie weist im Querschnitt entlang der Hauptströmungsrichtung H der Luft eine geschwungene Form auf, welche sich radial nach außen verbiegt und dabei deren Steigung laufend zunimmt. Alternativ kann sie auch im Wesentlichen flach ausgeführt sein, wobei sie besonders vorzugsweise in einem Winkel zum horizontalen Flüssigkeitsfilm steht. Dabei nähert sich die Leitfläche 14 vorzugsweise in Richtung der Hauptströmungsrichtung H in einem ersten Abschnitt zuerst an den Unterteil 2 des Radiallüfters an und entfernt sich danach in einem zweiten Abschnitt wieder. Dies führt zu einer Rundung im äußeren Bereich der Vorrichtung und auch im Bereich des Gasauslasses, aus dem die Luft ausströmt, wodurch Verwirbelungen an den Rändern verhindert werden. Die Luft entschleunigt und entstehendes Wasser rinnt an der Leitfläche 14 nach innen ab. Alternativ kann sich die Leitfläche 14 auch linear in einem ersten Abschnitt entlang der Hauptströmungsrichtung H zuerst dem Unterteil 2 annähern und sich nach einer Kante in einem zweiten Abschnitt von dem Unterteil 2 wieder entfernen oder weiters alternativ durchgehend nur entfernen, vorzugsweise linear.

Bei Inbetriebnahme der Vorrichtung 1 werden der Radiallüfter und damit das Ansaugstück 8 in Rotation versetzt. Durch den entstehenden Unterdruck wird die zu reinigende Luft durch den Lufteinlaufstutzen 3 angesaugt. Die Luft wird dabei durch das Ansaugstück 8 und den Radiallüfter geleitet.

Gleichzeitig wird über eine Spritzdüse (nicht gezeigt), Reinigungsflüssigkeit, bevorzugterweise Wasser aus der Leitung über einen Druckminderer im Wesentlichen senkrecht nach oben in den Zwischenraum 82 von Ansaugstück 8 und im Wesentlichen beabstandet parallel dazu verlaufende Form 81 (Hyperbelast - im Schnitt), wo, vorzugsweise hyperbolisch paraboloid gekrümmte Ansaugschaufeln 39, die die Wassersäule aufnehmen, beschleunigen und rundum einen gleichmäßigen Flüssigkeitsfilm 12 bilden. Alternativ können die Ansaugschaufeln 39 auch im Wesentlichen flach ausgeführt sein, wobei vorgesehen sein kann, dass sie nur im Bereich der oberen Kante 31 paraboloid ausgeführt sind.

Die Abtropfelemente 11 stehen dabei in einem Winkel w zum Flüssigkeitsfilm 12 von etwa 135°. Vorzugsweise ist der Winkel w in einem Bereich zwischen 130° und 140°.

Gleichzeitig wird auch mittels einer Pumpe über eine Spritzdüse (nicht gezeigt) Reinigungsflüssigkeit, bevorzugterweise Wasser, aus dem Flüssigkeitsbehälter 4 in den Zwischenraum 82 des rotierenden Ansaugstücks 8 eingebracht. Die eingespritzte Flüssigkeit wird erst aufgeschaufelt und aufgrund der herrschenden Fliehkräfte beschleunigt und weggeschleudert, bildet einen horizontalen Flüssigkeitsfilm 12, beginnend bei der Ansaugkörper 81 des Radiallüfters. Die Menge an angesaugter Luft wird hierbei über die Leistung des Radiallüfters gesteuert, wobei in Abhängigkeit von der Rotationsgeschwindigkeit des Ansaugstücks 8 auch die Eigenschaften des Flüssigkeitsfilms 12 beeinflusst werden.

Die angesaugte Luft tritt aus dem Radiallüfter aus und wird mittels Trimmring 9 durch den Wasserfilm 12 geleitet. Die Luft strömt hierbei an dem Trimmring 9, dessen Oberflächenneigung zum Unterteil 2, entlang und tritt anschließend erstmals durch den Wasserfilm 12 hindurch, wobei Luft in einem spitzen Winkel von oben nach unten ringförmig den Wasserfilm 12 flächig durchtritt. die horizontalen Wasserteilchen kollidieren an dieser Schnittfläche mit Verunreinigungen aus der Luft und werden von den Wasserteilchen absorbiert, dadurch verändert sich auch die Masse der Staubteilchen wesentlich. Durch die Zwangsführung der Luft wird erreicht, dass im nächsten Schritt Verunreinigungen direkt im Wassertank 4 landen oder an der nassen Innenseite der Wanne (Unterteil 2). In weiterer Folge wird die Luft erneut von (unten nach oben) großflächig durch den Wasserfilm geführt, um abermals von Wasserteilchen getroffen zu werden um noch verbleibende Staubteilchen zu binden. Im letzten Schritt der verfahrenstechnischen Anlage wird Luft von Wasserteilchen über eine Abtropfeinrichtung 10 wieder getrennt und entschleunigt und ringförmig über einen breiten Rand ausgeströmt. Durch die entropische Wirkung kühlt sich die Luft bei diesem Prozess um etwa 1 Kelvin ab. Da die Luft bei ihrem Durchtritt durch den Wasserfilm 12 Wassertropfen mitreißt, werden diese nun an den Borsten der Abtropfeinrichtung 10 mitsamt Verunreinigungen abgestreift, wobei das von der Abtropfeinrichtung 10 zurückgehaltene Wasser über die geneigt ausgeführte Oberfläche 13 des Unterteils 2 in den Flüssigkeitsbehälter 4 zurückfließt, von wo aus es wieder auf die oben beschriebene Weise in den Zwischenraum 82 von Ansaugstück 8 aufgebracht wird. Oberfläche 13 ist von der Abtropfeinrichtung 10 ca. 10 mm beabstandet. Das verunreinigte Wasser tropft ab und um keine Staubpartikel und Fasern zu hinterlassen, sind die Borsten von Oberfläche 13 beabstandet.

Mit anderen Worten tritt die angesaugte Luft aus dem Radiallüfter aus und wird mittels Trimmring 9 durch den Wasserfilm 12 geleitet. Die Luft strömt hierbei an dem Trimmring 9, dessen Oberflächenneigung zum Unterteil 2, entlang und tritt anschließend erstmals durch den Wasserfilm 12 hindurch, wobei zumindest ein Teil der in der Luft mitgeführten Verunreinigungen, insbesondere Rauch und Staub, an das Wasser abgegeben werden. Danach strömt die Luft entlang der entgegengesetzt zu dem Trimmring 9 geneigten Oberfläche 13 des Unterteils 2 und durchtritt abermals den Wasserfilm 12. Die nun mit Wasser angereicherte Luft gelangt schließlich zu der Abtropfeinrichtung 10, bevor sie die Vorrichtung 1 gereinigt, ionisiert und befeuchtet verlässt. Da die Luft bei ihrem Durchtritt durch den Wasserfilm 12 Wassertröpfchen mitreißt, werden diese nun an den Borsten 11 der Abtropfeinrichtung 10 mitsamt Verunreinigungen abgestreift, wobei das von der Abtropfeinrichtung 10 zurückgehaltene Wasser über die geneigt ausgeführte Oberfläche 13 des Unterteils 2 in den Flüssigkeitsbehälter 4 zurückfließt, von wo aus es wieder auf die oben beschriebene Weise auf das Ansaugstück 8 aufgebracht wird und abgepumpt werden kann oder auf die oben beschriebene Weise erneut in den Zwischenraum 82 von Ansaugstück 8 aufgebracht wird.

Die Borsten 11 sind in dem gezeigten Beispiel der Erfindung in Richtung der Luftströmung geneigt und an dem Oberteil 5 (Haube) befestigt. Sie können jedoch auch völlig vertikal ausgerichtet sein. Ebenso ist eine Variante denkbar, bei der eine Lage von Borsten 11 an dem Oberteil 5 befestigt und in Strömungsrichtung geneigt ist, während weitere, an dem Unterteil 2 befestigte Borsten 11 entgegen der Luftströmung geneigt sind. Im zusammengesetzten Zustand der Vorrichtung 1 sind die beiden Lagen ineinander verschränkt, sodass eine besonders dichte und effiziente Abtropfeinrichtung 10 erhalten wird, was besonders vorteilhaft für den Einsatz von Reinigungsflüssigkeiten ist, die nicht wässriger Natur sind, weil damit praktisch kein Lösungsmittel die erfindungsgemäße Vorrichtung verlassen kann.

Bei längerem Betrieb reichern sich die Verunreinigungen in dem Flüssigkeitsbehälter 4 aufgrund des oben beschriebenen Flüssigkeitskreislaufes an. Daher wird in regelmäßigen Abständen die Reinigungsflüssigkeit im Flüssigkeitsbehälter 4 getauscht. Den Verschmutzungsgrad kann mittels Sensoren ermittelt werden. Alternativ hierzu kann eine im Unterteil 2 angeordnete Ablasseinrichtung vorgesehen sein, die eine Entleerung des Flüssigkeitsbehälters 4 vor dem Öffnen der erfindungsgemäßen Vorrichtung 1 (Aufhängung) erlaubt.

Es versteht sich, dass die vorliegende Erfindung sich nicht auf die oben beschriebene Ausführung beschränkt ist. So kann beispielsweise ein eigener Auffangbehälter für die verunreinigte Reinigungsflüssigkeit vorgesehen sein. Ebenso kann die Abtropfeinrichtung andere Abtropfelemente anstatt der Borsten, wie beispielsweise Prallbleche aufweisen. Wesentlich ist jedoch, dass diese im Wesentlichen vertikal zum Flüssigkeitsfilm ausgerichtet sind und von Ebene 13 beabstandet.

Die dargestellte Ausführungsform hat einen Wasserverbrauch von etwa 5 l/h. Durch zumindest einen Zulauf wird Frischwasser zugeführt, durch zumindest einen mit Schwimmer geregelten Ablauf wird das Wasser über eine Pumpe abgepumpt, um so mit dem Wasser die sich im Wasser sammelnden Schmutzpartikel und Staubpartikel abzuführen.

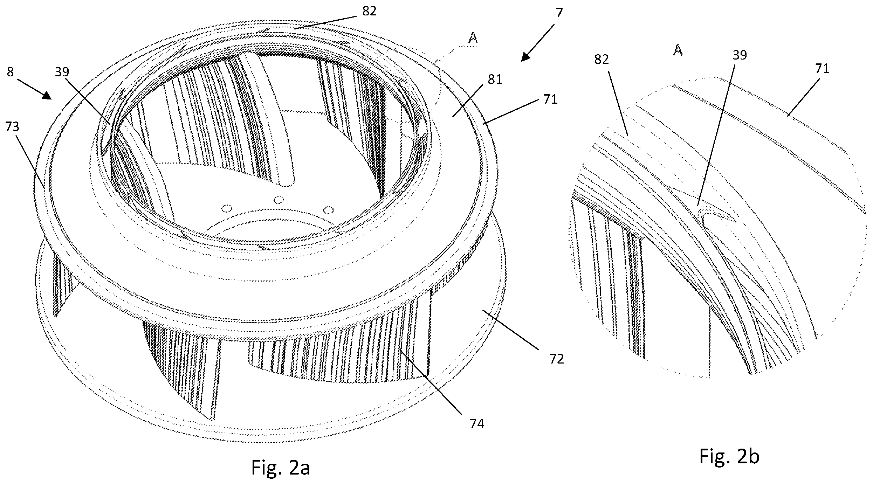

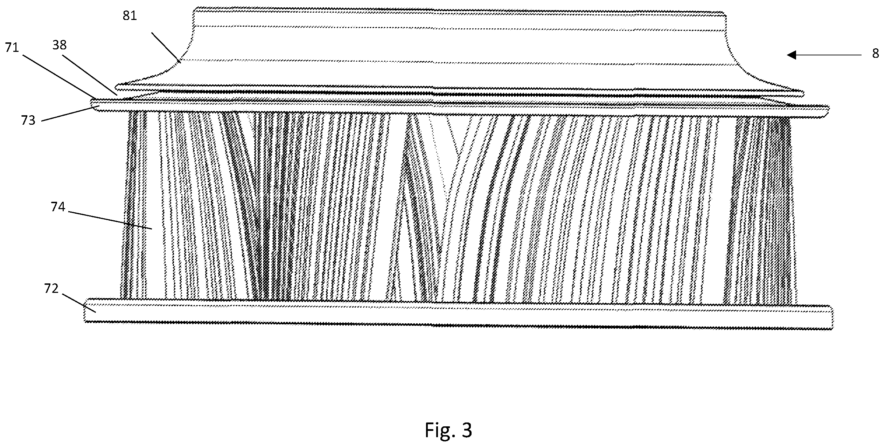

In Fig. 2a, 2b und 3 wird die Luftansaugeinrichtung 7 als Radiallüfter näher gezeigt. Dabei besitzt der Radiallüfter einen oberen Ringkörper 72 und einen unteren Ringkörper 73, zwischen welchen sechs Rotorblätter 74 gleichmäßig verteilt sind. Während der obere Ringkörper 72 im Wesentlichen flach ist, ist der untere Ringkörper 73 geschwungen und weist im Querschnitt die Form eines Hyperbelasts auf. Ein in der Form dem unteren Ringkörper 73 korrespondierender, ringförmiger und geschwungener Ansaugkörper 81 des Ansaugstücks 8 bildet den Zwischenraum 82 mit dem unteren Ringkörper 73. Im Zwischenraum 82 sind Ansaugschaufeln 39 angeordnet, die in sich doppelt verdreht sind. Dadurch wird das eintretende Wasser über den Umfang des Ansaugstücks 8 gleichmäßig verteilt und tritt als ringförmiger Wasserfilm 12, am ringförmigen geschwungenen Ansaugkörper 81, an der dem oberen Ringkörper 72 zugewandten Seite aus. Eine derartige Ausführungsform kann einen stabilen Flüssigkeitsfilm bei einer Rotation im Betrieb ab etwa 800 bis 1400 Umdrehungen pro Minute und darüber hinaus (z.B. 3000 Umdrehungen), aufbauen. Dadurch wird eine starke Geräuschbelastung vermieden, trotz hohem Luftdurchsatz. In der Einer bevorzugten Variante liegt die Umdrehungszahl der Luftansaugeinrichtung 7 bei etwa 1400 Umdrehungen und/oder der Luftdurchsatz der Vorrichtung bei etwa 1300 m³/h.

Das Wasser wird mittels Druckminderer über eine oder mehrere Düsen in den Zwischenraum 82 gespritzt, von wo es über den Umfang des Ansaugstücks 8 verteilt wird und bei Austritt den Wasserfilm 12 bildet. Weiters besteht die Möglichkeit die Wassertemperatur zu Regeln das wiederum austretende Lufttemperatur und Feuchte beeinflusst.

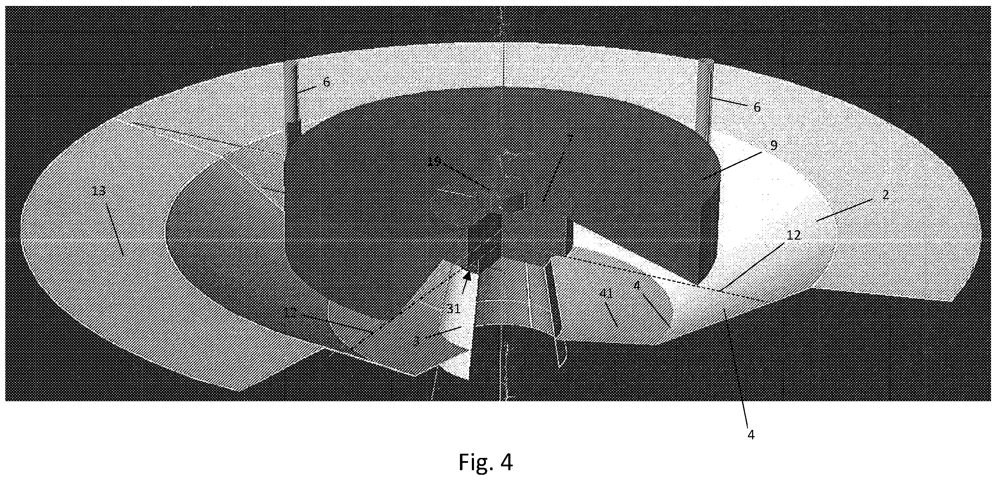

In Fig. 4 wird eine zweite erfindungsgemäße Ausführungsform gezeigt, die der ersten in weiten Teilen ähnelt. Daher wird hier nur auf die wichtigsten Unterschiede eingegangen. Sie wird in einer geschnittenen schematischen Seitenansicht dargestellt, wobei die Luftansaugeinrichtung 7 nur symbolisch als Block dargestellt ist. Die Ausführungsform weist einen Flüssigkeitsbehälter 4 auf, der einen schwach geneigten Boden 41 aufweist. Dies erhöht die aufnehmbare Wassermenge. Darüber hinaus ist sichtbar, dass die Luftumlenkeinrichtung 9 hohl ist, um möglichst leicht zu sein und einen Torus mit im Wesentlichen dreieckiger Rotationsfläche.

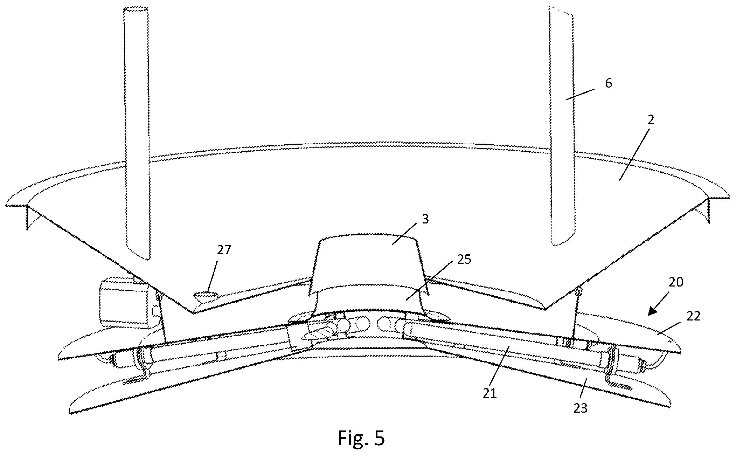

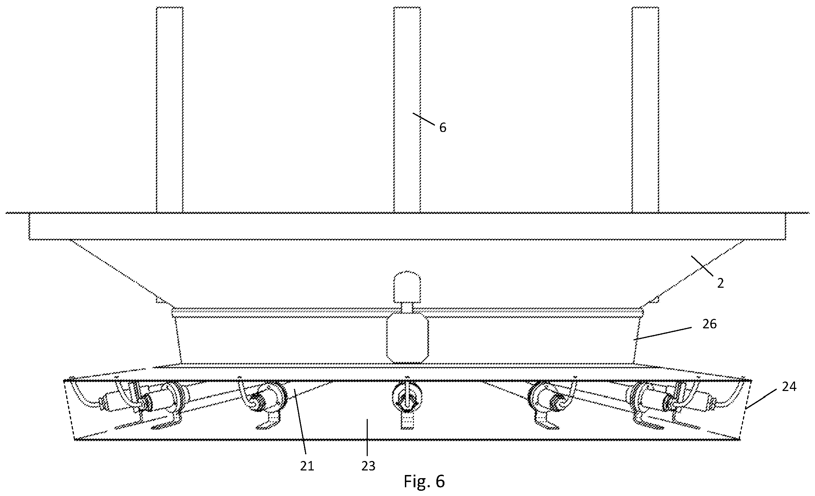

In Fig. 5 und 6 wird eine dritte Ausführungsform dargestellt, welche wie die erste Ausführungsform eine am Unterteil 2 angeordnete Bestrahlungseinheit 20 aufweist, in der insgesamt 12 UV-Lichtquellen 21 in Form von Leuchtstoffröhren angeordnet sind. Die Bestrahlungseinheit 20 weist eine Oberplatte 22 und eine Unterplatte 23 vorzugsweise aus Aluminium oder Stahl auf, zwischen denen die UV-Lichtquellen 21 angeordnet sind. Die Oberplatte 22 weist in ihrem Zentrum ein Zulaufteil 25 mit einer Öffnung auf, welches zum Lufteinlaufstutzen 3 gerichtet ist und die Luft in diesen einführt. Damit ist die Bestrahlungseinheit 20 stromaufwärts des Lufteinlaufstutzen 3 angeordnet.

Dabei kann die Verbindung zwischen Zulaufteil 25 und Lufteinlaufstutzen 3 dicht sein, womit ausschließlich Luft aus der Bestrahlungseinheit 20 in den Lufteinlaufstutzen 3 geführt wird. An der seitlichen Öffnung ist eine Filterwand 24 aus Vlies oder einem anderen Gewebe vorgesehen, welche luftdurchlässig ist, jedoch verhindert, dass das UV-Licht nach außen tritt. Durch diese Filterwand 24 wird die Luft angesaugt, erstmals grob gefiltert und danach am Weg zum Radiallüfter durch das UV-Licht bestrahlt. Die UV-Lichtquellen sind radial um die Luftansaugeinrichtung 7 angeordnet. Ein Verbindungsring 26 verbindet die Bestrahlungseinheit 20 mit dem Unterteil 2.

Die UV-Lichtquellen 21 erreichen zusammen vorzugsweise eine Leistung von 200 Watt. In der gezeigten Ausführungsform handelt es sich um 12 Leuchtstoffröhren mit einer Leistung von je 18 Watt.

Die Versorgungskabel der UV-Lichtquellen 21 können in den Befestigungshülsen 6 angeordnet werden, wo sie geschützt sind und nicht stören. Dies betrifft ebenso einen eventuell vorgesehenen Wasserzulauf oder Wasserablauf. Ein derartiger Wasserablauf 27 ist auch in der dritten Ausführungsform vorgesehen.

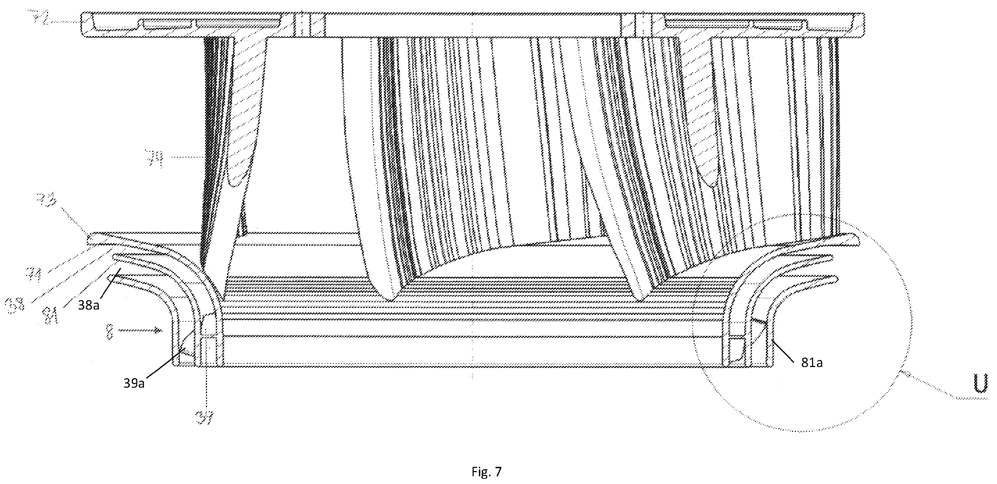

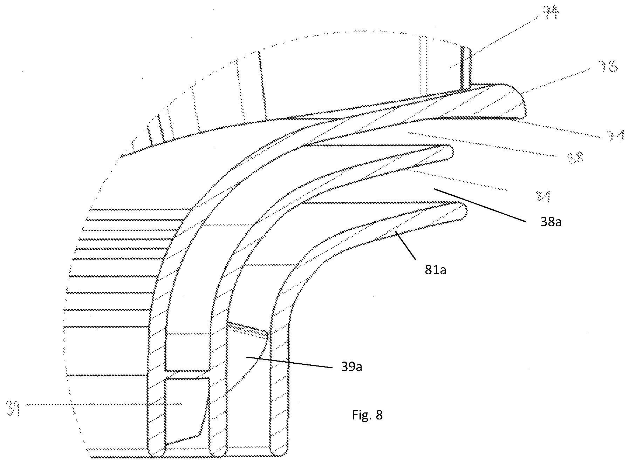

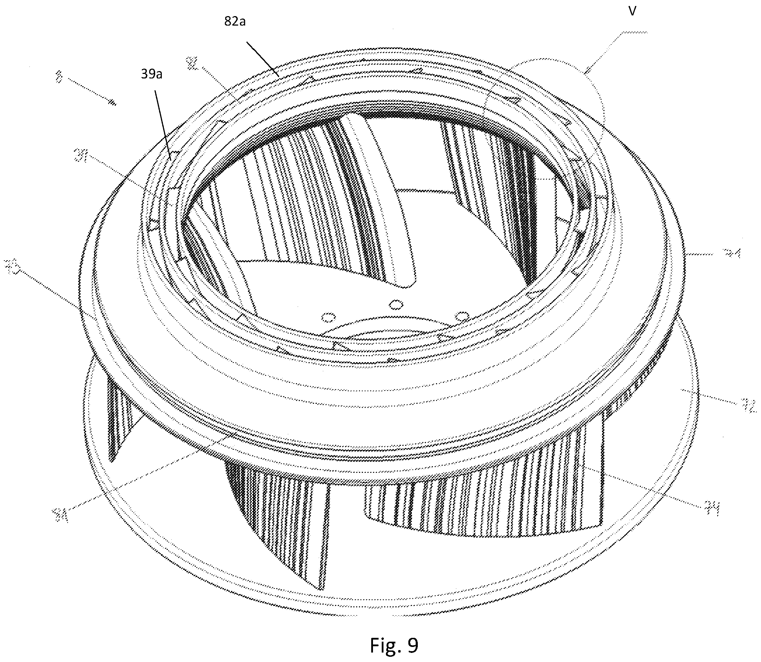

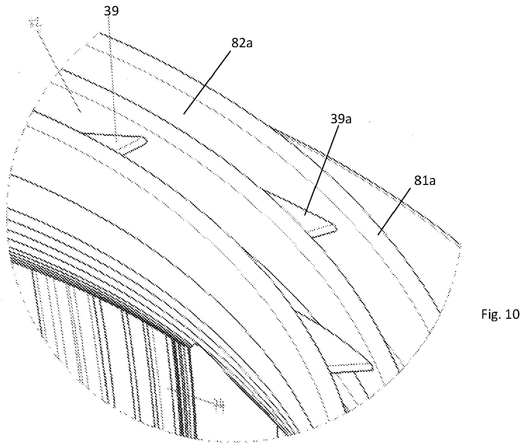

Die in den Figuren 7 bis 10 gezeigte alternative Ausführungsform der Luftansaugeinrichtung 7 als Radiallüfter ist der Ausführung der Figuren 2a, 2b und 3 sehr ähnlich, daher wird hier nur auf die wichtigsten Unterschiede eingegangen. Diese Ausführung weist zwei konzentrisch aneinander geordnete Ansaugkörper 81, 81a auf, welche hyperbelastartig an die geschwungene Form des unteren Ringkörpers angepasst sind und durch Auslassspalten 38, 38a jeweils einen stabilen Flüssigkeitsfilm abgeben. Damit sind die Flüssigkeitsfilme übereinander angeordnet und es müssen beide von der Luft passiert werden. Dies steigert den reinigenden Effekt. In beiden sich ergebenden Zwischenräumen 82, 82a sind dazu Ansaugschaufeln 39, 39a angeordnet, welche das Wasser in Richtung der Auslassspalten 38, 38a führen.

## Patentansprüche

1. Vorrichtung (1) zur Reinigung von Gas, insbesondere von Luft, mit einem Oberteil (5) und einem Unterteil (2), die miteinander in lösbarer Verbindung stehen, wobei in dem Unterteil (2) ein zentraler, vertikal angeordneter Lufteinlaufstutzen (3) für verunreinigte Luft vorgesehen ist, sowie ein Flüssigkeitsbehälter (4) für eine Reinigungsflüssigkeit mit einer Düseneinrichtung, und in dem Oberteil (5) eine Luftansaugeinrichtung (7) sowie eine Einrichtung zur Bildung zumindest eines horizontalen Flüssigkeitsfilms (12) und eine in dem Oberteil (5) angeordnete Luftumlenkeinrichtung, die die angesaugte Luft durch den Flüssigkeitsfilm (12) leitet, vorgesehen ist, **dadurch gekennzeichnet, dass** in einem äußeren Randbereich der Vorrichtung (1) zwischen Oberteil (5) und Unterteil (2) zumindest eine Abtropfeinrichtung (10) vorgesehen sind, deren Abtropfelemente (11) aus einer Vielzahl von Borsten bestehen und in einem Winkel (w) von maximal 160° zu dem horizontalen Flüssigkeitsfilm (12) ausgerichtet sind.

2. Vorrichtung (1) nach Anspruch 1, **dadurch gekennzeichnet, dass** die Einrichtung zur Bildung zumindest eines horizontalen Flüssigkeitsfilms (12) zumindest ein an der dem Lufteinlaufstutzen (3) zugewandten Seite der Luftansaugeinrichtung (7) angeordnetes Ansaugstück (8) aufweist, das bei Rotation der Luftansaugeinrichtung (7) ebenfalls rotiert.

3. Vorrichtung (1) nach Anspruch 2, **dadurch gekennzeichnet, dass** der Durchmesser des dem Ansaugstück (8) zugewandten Ende jenem des Lufteinlaufstutzens (3) entspricht und sich in Richtung Luftansaugeinrichtung (7) verbreitert, wobei die Kontur der Außenfläche von Ansaugstück (8) im Wesentlichen einem Hyperbelast entspricht, und der horizontale Flüssigkeitsfilm (12) an der Kante (81) zwischen Luftansaugeinrichtung (7) und Ansaugstück (8) entspringt.

4. Vorrichtung (1) nach einem der Ansprüche 1 bis 3, **dadurch gekennzeichnet, dass** der Flüssigkeitsbehälter (4) im Unterteil (2) um den Lufteinlaufstutzen (3) spitz zusammenläuft.

5. Vorrichtung (1) nach einem der Ansprüche 1 bis 4, **dadurch gekennzeichnet, dass** die Luftansaugeinrichtung (7) ein Radiallüfter ist.

6. Vorrichtung (1) nach einem der Ansprüche 1 bis 5, **dadurch gekennzeichnet, dass** die Luftumlenkeinrichtung (9) über zu dem horizontalen Flüssigkeitsfilm (12) hin abfallende, vorzugsweise trimmbare Leitbleche verfügt, die die angesaugte Luft durch den Flüssigkeitsfilm (12) durchleiten.

7. Vorrichtung (1) nach einem der Ansprüche 1 bis 6, **dadurch gekennzeichnet, dass** die Abtropfelemente (11) aus einer Vielzahl von Borsten (11) gebildet sind.

8. Vorrichtung (1) nach einem der Ansprüche 1 bis 7, **dadurch gekennzeichnet, dass** die Abtropfeinrichtung (10) mit dem Flüssigkeitsbehälter (4) in Verbindung steht, sodass die in der Abtropfeinrichtung (10) abgeschiedene, Verunreinigungen enthaltende Flüssigkeit in den Flüssigkeitsbehälter (4) zurückfließt.

9. Vorrichtung (1) nach einem der Ansprüche 1 bis 8, **dadurch gekennzeichnet, dass** die Abtropfeinrichtung (10) zumindest eine Leitfläche (14) aufweist, welche entlang einer Hauptströmungsrichtung (H) eine geschwungene Form mit aufweist, wobei die Leitfläche (14) entlang der Hauptströmungsrichtung (H) von vom Gasstrom weg biegt.

10. Vorrichtung (1) nach einem der Ansprüche 1 bis 9, **dadurch gekennzeichnet, dass** die Reinigungsflüssigkeit Wasser ist.

11. Vorrichtung (1) nach einem der Ansprüche 1 bis 10, **dadurch gekennzeichnet, dass** die Vorrichtung (1) zumindest eine UV-Lichtquelle (21) zur Bestrahlung der Luft aufweist.

12. Vorrichtung (1) nach Anspruch 11, **dadurch gekennzeichnet, dass** eine die UV-Lichtquelle (21) umfassende Bestrahlungseinheit (20) an der, dem Oberteil (5) abgewandten Seite des Unterteils (2) angeordnet ist.

13. Vorrichtung (1) nach Anspruch 12, **dadurch gekennzeichnet, dass** die Bestrahlungseinheit (20) eine gasdurchlässige, UV-lichtdichte Filterwand (24) aufweist.

14. Verfahren zur Reinigung von Luft unter Verwendung einer Vorrichtung (1) nach einem der Ansprüche 1 bis 9, **dadurch gekennzeichnet, dass** eine Flüssigkeit aus einem Flüssigkeitsbehälter (4) horizontal verteilt wird, die zu reinigende Luft zumindest einmal durch den horizontalen Flüssigkeitsfilm (12) hindurchgeleitet wird, die Luft nach dem Durchtritt durch den Flüssigkeitsfilm (12) über eine Abtropfeinrichtung (10) geführt wird, und die in der Luft befindliche, mit Verunreinigungen der Luft angereicherte Flüssigkeit an in einem Winkel (w) von maximal 160° zu dem horizontalen Flüssigkeitsfilm (12) angeordneten Abtropfelementen (11) der Abtropfeinrichtung (10), die aus einer Vielzahl von Borsten bestehen, abgeschieden wird.

15. Verfahren nach Anspruch 14, **dadurch gekennzeichnet, dass** die Luft mit UV-Licht zumindest einer UV-Lichtquelle (21) bestrahlt wird, vorzugsweise bevor sie durch den Flüssigkeitsfilm (12) hindurchgeleitet wird.

## Claims

1. Device (1) for cleaning gas, in particular air, having an upper part (5) and a lower part (2) which are connected to each other in a detachable manner, wherein a central, vertically arranged air inlet nozzle (3) for contaminated air is provided in the lower part (2), as well as a liquid container (4) for a cleaning liquid with a nozzle device, and an air suction device (7) and a device for forming at least one horizontal liquid film (12) and an air deflection device arranged in the upper part (5), which directs the sucked-in air through the liquid film (12), are provided in the upper part (5), **characterised in that** at least one drip unit (10) is provided in an outer edge area of the device (1) between the upper part (5) and the lower part (2), the drip elements (11) of which consist of a plurality of bristles and are aligned at an angle (w) of at most 160° to the horizontal liquid film (12).

2. Device (1) according to claim 1, **characterised in that** the device for forming at least one horizontal liquid film (12) has at least one suction piece (8) which is arranged on the side of the air suction device (7) facing the air inlet nozzle (3) and which also rotates when the air suction device (7) rotates.

3. Device (1) according to claim 2, **characterised in that** the diameter of the end facing the suction piece (8) corresponds to that of the air inlet nozzle (3) and widens in the direction of the air suction device (7), wherein the contour of the outer surface of the suction piece (8) essentially corresponds to a hyperbola, and the horizontal liquid film (12) originates at the edge (81) between the air suction device (7) and the suction piece (8).

4. Device (1) according to one of claims 1 to 3, **characterised in that** the liquid container (4) converges pointedly in the lower part (2) around the air inlet nozzle (3).

5. Device (1) according to one of claims 1 to 4, **characterised in that** the air suction device (7) is a radial fan.

6. Device (1) according to one of claims 1 to 5, **characterised in that** the air deflection device (9) has guide plates which slope down toward the horizontal liquid film (12), are preferably trimmable and guide the sucked-in air through the liquid film (12).

7. Device (1) according to one of claims 1 to 6, **characterised in that** the drip elements (11) are formed from a plurality of bristles (11).

8. Device (1) according to one of claims 1 to 7, **characterised in that** the drip unit (10) is connected to the liquid container (4) so that the liquid containing impurities separated in the drip unit (10) flows back into the liquid container (4).

9. Device (1) according to one of claims 1 to 8, **characterised in that** the drip unit (10) has at least one guide surface (14) which has a curved shape along a main flow direction (H), wherein the guide surface (14) curves away from the gas flow along the main flow direction (H).

10. Device (1) according to one of claims 1 to 9, **characterised in that** the cleaning liquid is water.

11. Device (1) according to one of claims 1 to 10, **characterised in that** the device (1) has at least one UV light source (21) for irradiating the air.

12. Device (1) according to claim 11, **characterised in that** an irradiation unit (20) comprising the UV light source (21) is arranged on the side of the lower part (2) facing away from the upper part (5).

13. Device (1) according to claim 12, **characterised in that** the irradiation unit (20) has a gas-permeable, UV-light-tight filter wall (24).

14. Method for cleaning air using a device (1) according to one of claims 1 to 9, **characterised in that** a liquid is distributed horizontally from a liquid container (4), the air to be cleaned is passed at least once through the horizontal liquid film (12), the air is guided over a drip unit (10) after passing through the liquid film (12), and the liquid in the air, enriched with air contaminants, is separated by drip elements (11) of the drip unit (10), which are arranged at an angle (w) of at most 160° to the horizontal liquid film (12) and consist of a plurality of bristles.

15. Method according to claim 14, **characterised in that** the air is irradiated with UV light from at least one UV light source (21), preferably before it is passed through the liquid film (12).

## Revendications

1. Installation (1) d'épuration de gaz, en particulier d'air, comprenant une partie (5) supérieure et un partie (2) inférieure, qui sont assemblées l'une à l'autre, de manière amovible, dans laquelle il est prévu dans la partie (2) inférieure, pour de l'air non épuré, une tubulure (3) centrale d'entrée d'air disposée verticalement, ainsi qu'un récipient (4) à liquide pour un liquide d'épuration ayant un dispositif de buses, et dans la partie (5) supérieure est prévue au moins un dispositif (7) d'aspiration d'air, ainsi qu'un dispositif de formation d'au moins une pellicule (12) horizontale de liquide et un dispositif de déviation d'air, disposé dans la partie (5) supérieure et qui fait passer l'air aspiré à travers la pellicule (12) de liquide, **caractérisée en ce que** dans une zone marginale extérieure de l'installation (1), il est prévu, entre la partie (5) supérieure et la partie (2) inférieure, au moins un dispositif (10) d'égouttage, dont les éléments (11) d'égouttage sont constitués d'une pluralité de soies et sont dirigés en faisant un angle (w) de 160° au maximum avec la pellicule (12) horizontale liquide.

2. Installation (1) suivant la revendication 1, **caractérisée en ce que** le dispositif de formation d'au moins une pellicule (12) horizontale liquide a au moins une pièce (8) d'aspiration, qui est disposée du côté du dispositif (7) d'aspiration d'air, tourné vers la tubulure (3) d'entrée d'air, et qui tourne également lorsque le dispositif (7) d'aspiration d'air tourne.

3. Installation (1) suivant la revendication 2, **caractérisée** en ce le diamètre de l'extrémité, tournée vers la pièce (8) d'aspiration, correspond à celui de la tubulure (3) d'entrée d'air et s'élargit dans la direction du dispositif (7) d'aspiration d'air, dans laquelle le contour de la surface extérieure de la pièce (8) d'aspiration correspond sensiblement à une hyperbole et la pellicule (12) horizontale de liquide s'échappe du bord (81) entre le dispositif (7) d'aspiration d'air et la pièce (8) d'aspiration.

4. Installation (1) suivant l'une des revendications 1 à 3, **caractérisée en ce que** le récipient (4) à liquide pointe dans la partie (2) inférieure autour de la tubulure (3) d'entrée d'air.

5. Installation (1) suivant l'une des revendications 1 à 4, **caractérisée en ce que** le dispositif (7) d'aspiration d'air est un ventilateur radial.

6. Installation (1) suivant l'une des revendications 1 à 5, **caractérisée en ce que** le dispositif (9) de déviation d'air dispose de chicanes, pouvant, de préférence, être raccourcies, tombant vers la pellicule (12) horizontale de liquide et faisant passer l'air aspiré à travers la pellicule (12) de liquide.

7. Installation (1) suivant l'une des revendications 1 à 6, **caractérisée en ce que** les éléments (11) d'égouttage sont constitués d'une pluralité de soies (11).

8. Installation (1) suivant l'une des revendications 1 à 7, **caractérisée en ce que** le dispositif (10) d'égouttage est en communication avec le récipient (4) à liquide de sorte que le liquide contenant des impuretés, séparé dans le dispositif (10) d'égouttage, retourne au récipient (4) à liquide.

9. Installation (1) suivant l'une des revendications 1 à 8, **caractérisée en ce que** le dispositif (10) d'égouttage a au moins une surface (14) de conduite, qui a, suivant une direction (H) d'écoulement principal, une forme incurvée, dans laquelle la surface (14) de conduite se courbe suivant la direction (H) d'écoulement principal en s'éloignant du courant gazeux.

10. Installation (1) suivant l'une des revendications 1 à 9, **caractérisée en ce que** le liquide d'épuration est l'eau.

11. Installation (1) suivant l'une des revendications 1 à 10, **caractérisée en ce qu'**une installation (1) a au moins une source (21) lumineuse UV pour exposer l'air à du rayonnement.

12. Installation (1) suivant la revendication 11, **caractérisée en ce qu'**une unité (20) d'émission de rayonnement comprenant la source (21) lumineuse UV est disposée sur la face de la partie (2) inférieure, non tournée vers la partie (5) supérieure.

13. Installation (1) suivant la revendication 12, **caractérisée en ce que** l'unité (20) de rayonnement a une paroi (24) de filtre, perméable au gaz et étanche à la lumière UV.

14. Procédé d'épuration de l'air en utilisant une installation (1) suivant l'une des revendications 1 à 9, **caractérisé en ce que** l'on répartit horizontalement un liquide provenant d'un récipient (4) à liquide, on fait passer l'air épuré au moins une fois à travers la pellicule (12) horizontale de liquide, on envoie l'air après la traversé de la pellicule (12) de liquide sur un dispositif (10) d'égouttage et on sépare le liquide, se trouvant dans l'air et enrichi des impuretés de l'air, sur des éléments (11) d'égouttage du dispositif (10) d'égouttage, qui font un angle (w) de 160° au maximum avec la pellicule (12) horizontale de liquide et qui sont constitués d'une pluralité de soies.

15. Procédé suivant la revendication 14, **caractérisé en ce que** l'on expose l'air à de la lumière UV d'au moins une source lumineuse (21) UV, de préférence avant qu'il ne passe à travers la pellicule (12) de liquide.
